# EUROPEAN PATENT APPLICATION

(11) **EP 3 869 511 A1**
(43) Date of publication of application: **25.08.2021**
(21) Application number: 19873589.6
(22) Date of filing: 15.10.2019
(51) Int. Cl.: G16B 40/00

(54) **METHOD AND SYSTEM FOR ANNOTATING SCOPE OF CLAIMS OF GENE SEQUENCE, METHOD AND SYSTEM FOR SEARCHING GENE SEQUENCE, AND METHOD AND SYSTEM FOR ANNOTATING INFORMATION OF GENE SEQUENCE**

(30) Priority: 15.10.2018 CN 201811197975
(71) Applicant: Patsnap Limited, Jiangsu 215000 (CN)
(72) Inventor: KUMAR, Pankaj, Suzhou Industrial Park, Jiangsu 215000 (CN); CAI, Jie, Suzhou Industrial Park, Jiangsu 215000 (CN); HAENSE, Markus, Suzhou Industrial Park, Jiangsu 215000 (CN); HUSSEIN, Ali, Suzhou Industrial Park, Jiangsu 215000 (CN)
(74) Representative: Slingsby Partners LLP
(86) International application number: PCT/CN2019/111146
(87) International publication number: WO 2020/078341

(57) **Abstract**

Embodiments of the present disclosure provides a method and a system for labeling and retrieving the protection scope of claims and for labeling information of a gene sequence, wherein the method includes: recognizing a gene sequence from the claims of the current patent application; extracting descriptive texts of the gene sequence from the claims based on a preset keyword; determining similarity information of the gene sequence based on the extracted descriptive texts, and labeling the scope of the claims of the gene sequence based on the similarity information. In the technical solutions provide in the embodiments of the present disclosure, a sequence retrieval can be performed in a patent library, and the accuracy of the gene sequence retrieval can be improved.

## Description

### CROSS-REFERENCE TO RELATED APPLICATIONS

This application claims priority to Chinese Patent Application No. 201811197975.2, entitled "Method and System for Claim Scope Labeling, Retrieval and Information Labeling of Gene Sequence", filed on October 15, 2018, both of which are hereby incorporated by reference in their entireties.

### TECHNICAL FIELD

Embodiments of the present disclosure relate to the field of Internet technology, and in particular to methods and systems for scope of claims labeling, retrieval and information labeling of a gene sequence.

### BACKGROUND

At present, when retrieving a gene sequence in the biological field, a retrieval engine usually receives a gene sequence to be retrieved from a user, then compares the gene sequence to be retrieved with gene sequences in a gene bank, and queries out a relevant gene sequence from the gene bank.

### SUMMARY

It is an object of the embodiments of the present disclosure aim to provide a method and a system for labeling and retrieving the protection scope of claims and for labeling information of a gene sequence, which can retrieve the gene sequence in a patent library.

In order to achieve the above object, some embodiments of this disclosure are implemented as follows.

A method for labeling a protection scope of claims of a gene sequence, characterized in that, the method is applied to a retrieval engine, the method comprising:
recognizing a gene sequence from claims of a current patent application;
extracting descriptive texts of the gene sequence from the claims according to a preset keyword; and
determining similarity information of the gene sequence based on the extracted descriptive texts, and labeling the protection scope of the claims of the gene sequence based on the similarity information.

A system for labeling a protection scope of claims of a gene sequence, characterized by comprising:
a gene sequence recognition unit for recognizing the gene sequence from the claims of a current patent application;
a descriptive text extraction unit for extracting descriptive texts of the gene sequence from the claims based on a preset keyword; and
a claim scope determination unit for determining similarity information of the gene sequence based on the extracted descriptive texts, and labeling the protection scope of the claims of the gene sequence based on the similarity information.

A method for retrieving a gene sequence, characterized by comprising:
acquiring a gene sequence to be retrieved;
grabbing a text gene sequence from patent applications in a patent library, and comparing the gene sequence to be retrieved with the grabbed text gene sequence, to determine a target gene sequence similar to the gene sequence to be retrieved; and
displaying a retrieval result of patent applications containing the target gene sequence.

A method for retrieving a gene sequence, characterized by comprising:
acquiring a gene sequence to be retrieved and a preset similarity threshold value;
splitting the gene sequence to be retrieved into a plurality of gene subsequences based on the preset similarity threshold value; and
performing a sequence retrieval for each of the gene subsequences in a patent library to determine a target patent application corresponding to the gene subsequence, and outputting a retrieval result of the target patent application.

A method for labeling infringement risk information of a gene sequence, characterized in that, the method is applied to a retrieval engine, and the method comprises:
acquiring a gene sequence to be retrieved, and querying patent applications containing a target gene sequence similar to the gene sequence to be retrieved;
extracting descriptive texts containing the target gene sequence from the claims of the patent applications, and determining a scope of the claims of the target gene sequence based on the descriptive texts;
determining a similarity between the gene sequence to be retrieved and the target gene sequence, and comparing the similarity with the scope of claims of the target gene sequence; and
labeling infringement risk information of the patent applications with respect to the gene sequence to be retrieved based on a comparison result.

As can be seen from the above, in one or more embodiments of the present disclosure, after a gene sequence to be retrieved provided by a user is acquired, the patent application(s) matching the gene sequence to be retrieved can be queried at first in accordance with the similarity between the gene sequences. Specifically, it is possible to grab a text gene sequence from the patent application(s) in a patent library, and to compare the gene sequence to be retrieved with the grabbed text gene sequence, to determine a target gene sequence similar to the gene sequence to be retrieved. The patent application(s) containing the target gene sequence can then be regarded as the patent application(s) which matches the gene sequence to be retrieved. In these patent applications, a target gene sequence similar to the gene sequence to be retrieved may be included. However, in scenarios such as infringement retrieval analysis, it is not sufficient to provide only a retrieval result of similarity of the gene sequences. Assuming that the user conducts an infringement retrieval analysis on a gene sequence, the patent application retrieved based on the similarity may have only a target gene sequence with a similarity of 90%, but have no completely consistent gene sequence. However, it can not be concluded that the gene sequence can be used freely at this time. The reason is that the target gene sequence contained in the retrieved patent application usually has a protection scope, this protection scope may cover, for example, a gene sequence having a similarity of 80% or more to the target gene sequence involved in the patent application. Thus, since the similarity between the gene sequence to be retrieved and the target gene sequence is 90%, which actually falls within the protection scope of the patent application, and thus there is still a possibility of infringement. In view of this, in the present disclosure, after the patent application is obtained by performing retrieval based on the similarity, descriptive texts containing the target gene sequence may be further extracted from the claims of the patent application, and a protection scope of the target gene sequence may be determined according to the descriptive texts. Then, the similarity corresponding to the patent application and the protection scope can be displayed together in the retrieval result, so that the user can compare the size relationship between the similarity and protection scope, so as to judge whether the gene sequence to be retrieved has the possibility of infringement. As can be seen from the above, the technical solution provided in one or more embodiments of the present disclosure can not only retrieve a gene sequence in the patent library, but also provide users with more abundant retrieval information, thereby improving the accuracy of the retrieval result.

### BRIEF DESCRIPTION OF DRAWINGS

To illustrate more clearly the technical solutions in one or more embodiments of the present disclosure or in the prior art, accompanying drawings required to be used in the embodiments or in the prior art will be briefly introduced below. Obviously, the accompanying drawings described below are merely some embodiments recorded in this disclosure. For those of ordinary skill in the art, other drawings can also be obtained from these drawings without creative efforts.
FIG. 1 is a flow chart of a method for labeling a protection scope of claims of a gene sequence provided in the present disclosure;
FIG. 2 is a schematic diagram of a method for recognizing a target gene sequence provided in the present disclosure;
FIG. 3 is a schematic diagram of functional modules of a system for labeling a protection scope of claims of a gene sequence provided in the present disclosure;
FIG. 4 is a structural schematic diagram of a system for labeling a protection scope of claims of a gene sequence provided in the present disclosure;
FIG. 5 is a schematic diagram of a method for retrieving a gene sequence provided in the present disclosure;
FIG. 6 is a picture displaying a retrieval result page provided in the present disclosure;
FIG. 7 is a schematic diagram of a similarity map of a gene sequence provided in the present disclosure;
FIG. 8 is a schematic diagram of a development progress map of a gene sequence provided in the present disclosure;
FIG. 9 is a schematic diagram of a method for retrieving a gene sequence provided in the present disclosure;
FIG. 10 is a schematic diagram of a split of a gene sequence provided in the present disclosure;
FIG. 11 is a flow chart of a method for labeling infringement risk information of a gene sequence provided in the present disclosure; and
FIG. 12 is a structural schematic diagram of a system for labeling infringement risk information of a gene sequence provided in the present disclosure.

### DESCRIPTION OF EMBODIMENTS

In order to make those skilled in the art better understand the technical solutions in the present disclosure, the technical solutions in the embodiments of the present disclosure are clearly and completely described in the following with reference to the accompanying drawings in the embodiments of the present disclosure. Obviously, the described embodiments are merely a part of, rather than all of the embodiments of the present disclosure. Based on the embodiments of the present disclosure, all other embodiments that are obtained by persons skilled in the art without making creative efforts shall fall within the protection scope of the present disclosure.

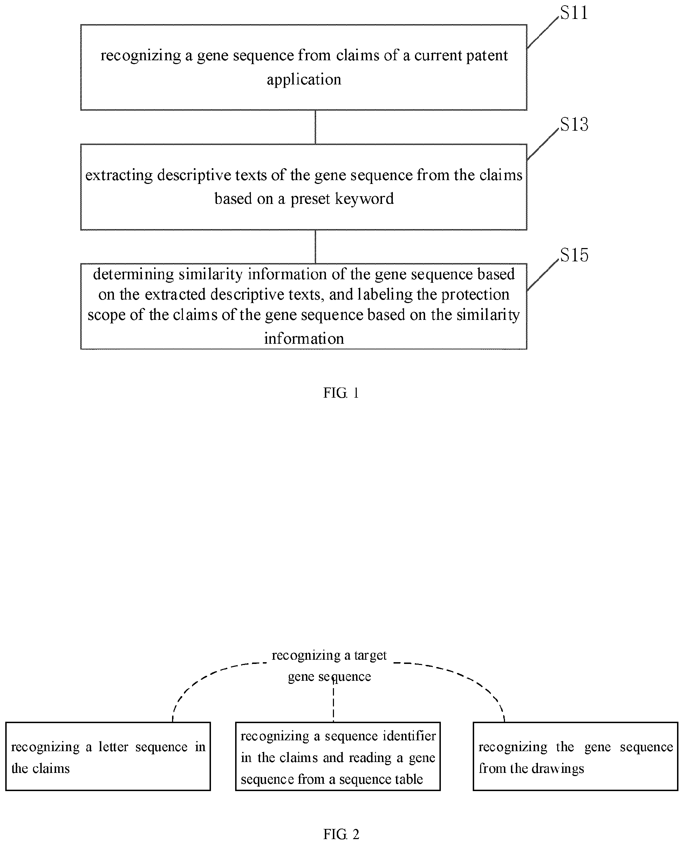

There is provided in the present disclosure a method for labeling a protection scope of claims of a gene sequence, which may be applied to a retrieval engine. Referring to FIG. 1, the method may comprise the following steps.

S11: recognizing a gene sequence from claims of a current patent application.

In the embodiments, for patent applications related to gene sequences that already exist in the patent library, the scope of claims of the gene sequence in each of the patent applications can be determined respectively. The current patent application may be a patent application to be analyzed, and the gene sequence recorded in the claims of the patent application to be analyzed may be used as a target gene sequence to be currently analyzed.

In the embodiments, the gene sequence can be recognized from the claims of the current patent application in a manner as shown in FIG. 2. Specifically, the gene sequence is usually a sequence of letters consisting of a number of abbreviated letters. For example, a letter A represents alanine, a letter R represents arginine, a letter H represents histidine, and the like. In this way, if there is a letter sequence consisting of successive letters in the claims, the letter sequence may possibly be the gene sequence. Therefore, a letter sequence consisted of the specified letters can be recognized from the claims of the current patent application by way of text matching. Herein, the specified letters are abbreviated letters of the gene sequence. In this way, after a letter sequence consisted of specified letters is recognized, it is considered that a gene sequence generally has a certain length. If the length of the letter sequence is short, it may be just ordinary English words or phrases. In view of this, it is possible to further judge the length of the letter sequence, and a letter sequence can be taken as a gene sequence when the length of the letter sequence reaches a specified length threshold. The specified length threshold may be an average value calculated by statistically calculating lengths of a huge number of gene sequences, or may be obtained by subtracting or adding a certain amount of redundancy on the basis of the average value.

In other embodiments, considering that in the claims of a patent application, the gene sequence is usually represented by a sequence identifier in order to satisfy the requirements of language brevity, and the real gene sequence is indicated in a sequence table. Therefore, in the embodiments, it is possible to recognize a sequence identifier from the claims of the current patent application, and to read a gene sequence corresponding to this sequence identifier from the sequence table of the current patent application. Herein, the sequence identifier may be a sequence number for characterizing a gene sequence, for example, the sequence identifier in the claims may an indicative identifier, such as "SEQ ID No. 1" and "SEQ ID No. 2". By means of text matching, such an indicative identifier can be recognized in the claims of the current patent application. Then the sequence table of the patent application can be read, and a gene sequence corresponding to the sequence identifier can be read from the sequence table.

Of course, it should be noted that the gene sequence in the patent application is sometimes labeled in the specification in a form of text, or labeled in the accompanying drawings in a form of drawing. Therefore, referring to FIG. 2, in practical application, for a gene sequence coming in the form of text, a letter sequence consisted of specified letters can be recognized, or the corresponding gene sequence can be read from the sequence table, by using the manner described in the above embodiments. For the gene sequence displayed in the form of the drawing, the character string included in the drawings can be recognized by using an OCR (Optical Character Recognition) technique, and the recognized character string may be used as a gene sequence.

S13: extracting descriptive texts of the gene sequence from the claims based on a preset keyword.

In the embodiments, the claims of the current patent application may be analyzed to extract descriptive texts containing the gene sequence from the claims. The descriptive texts may be used to define the scope of claims of the gene sequence. Specifically, the descriptive texts of the gene sequence can be extracted by performing a semantic analysis on the claims of the patent application by using a natural language processing (NLP) technique. Specifically, the retrieval engine may take a large number of claims describing gene sequences as training samples in advance, and train a natural language model by using machine learning, which can recognize the meanings characterized by different phrases in the claims and the associations among multiple phrases. During the training of the natural language model, firstly, text related to description of the protection scope of the gene sequence can be labeled in the training samples through manual labeling. For example, for the following training samples:

"A polypeptide structure comprising at least 70% of the amino acids in SEQ ID No. 2, or amino acids at positions 48 to 56, 90 to 101, and 161 to 169 in SEQ ID No. 4."

In these training samples, the cited gene sequences can be labeled. For example, the "SEQ ID No. 2" and "SEQ ID No. 4" in the above example can be labeled with seq_id. Moreover, the text used to define the length of a partial subsequence in the gene sequence may also be labeled. Herein, the text describing the length of the subsequence may include a percentage numerical value, such as "70%" as described above, and may also include regional information for defining the position of a subsequence in the gene sequence, such as "48 to 56," "90 to 101 " and "161 to 169 ", as described above. When the text describing the length of a subsequence is labeled, it can be labeled differently according to different types. For example, for a percentage numerical value, it can be labeled with "quantity"; and for regional information, it can be labeled with "region". Moreover, the text describing the length of the subsequence will typically be associated with the cited gene sequence, for example, "70%" in the above example is associated with SEQ ID No. 2, and the texts "48 to 56," "90 to 101" and "161 to 169" are associated with the SEQ ID No. 4. Therefore, when the manual labeling is performed, two objects that are associated with each other can also be labeled in a form of key-value. For example, the labeling result may be "70% - SEQ ID No. 2", "48 to 56, 90 to 101, 161 to 169 - SEQ ID No. 4". Of course, in practical application, the above-mentioned labeling manner can also be changed flexibly according to different rules, and the above exemplary labeling situation is only for describing the technical solution of this embodiment, which is not meant that the technical solution of the embodiments can be labeled only by the above labeling method. In this way, the labeled training samples can be input into the natural language model, and a neural network in the natural language model can learn the labeled learning samples, so as to distinguish the semantics of each sentence and the correlation between different sentences according to the way of presenting the claims. After training a large number of samples, the trained natural language model can recognize the claims that are not labeled, so as to recognize the gene sequence and the text describing the length of the subsequence therefrom, which are labeled at the training stage. In the subsequent analysis of the claims that are not labeled, the natural language model obtained by training can be used to recognize the labeling information corresponding to part of phrases in the claims, and the recognized labeling information can be used as a preset keyword for extracting the descriptive texts of the gene sequence from the claims.

In the embodiments, a trained natural language model may be integrated into a retrieval engine, so that for the current patent application, the position where the gene sequence is located may be recognized in the claims by using the natural language module. After the position where the gene sequence is located is recognized, a length identifier that defines the length of a partial subsequence in the gene sequence can be looked up from the context of the recognized position by using the natural language model. The length identifier can be the text used to describe the percentage and the regional information of the subsequence as mentioned above. In this way, the descriptive texts of the gene sequence can be obtained by taking the length identifier as a preset keyword and intercepting the sentence containing the length identifier and the gene sequence.

S15: determining similarity information of the gene sequence based on the extracted descriptive texts, and labeling a protection scope of the claims of the gene sequence based on the similarity information.

In the embodiments, after the descriptive texts containing the gene sequence are obtained by extraction, semantic analysis may be performed on the descriptive texts, so as to determine the scope of claims of the gene sequence in the descriptive texts. Specifically, if the extracted descriptive texts contain a percentage numerical value for describing the gene sequence, an effective percentage corresponding to the gene sequence can be directly recognized from the descriptive texts, and this effective percentage is taken as similarity information of the gene sequence. For example, in the above example, "70%" can be used as the similarity information of the SEQ ID No. 2. However, in some patent applications, the effective percentage of the gene sequence is not described directly, but the regional information of the subsequence therein is described, such as "48 to 56", "90 to 101" and "161 to 169", as described in the above example for the SEQ ID No. 4. In this case, regional information for defining a position of a gene subsequence or a core function sequence in the gene sequence can be recognized from the descriptive texts, and a length of the gene subsequence or the core function sequence can be determined according to the regional information. Herein, the gene subsequence may refer to a partial gene sequence located in the gene sequence, which is defined by the regional information. The core function sequence may also be a partial gene sequence which is defined by the regional information and is important in the gene sequence. For example, among many gene sequences in a human body, a portion of the nucleotide sequences can characterize a potential cancer risk in the body. In this case, these nucleotide sequences can be used as the core function sequence in the gene sequences. In the above example, the gene subsequence or the core function sequence is composed of three parts, a sum of lengths of which is 9 + 12 + 9 = 30. Then, a percentage of the length of the gene subsequence or the core function sequence in the total length of the gene sequence may be calculated, and the calculated percentage may be taken as the similarity information of the gene sequence. Assuming that the total length of SEQ ID No. 4 is 200, the calculated percentage is 30/200 = 15%, and therefore, the similarity information of SEQ ID No. 4 is 15%.

In the embodiments, after the similarity information of the gene sequence is determined, the scope of claims of the gene sequence in the claims takes the percentage numerical value represented by the similarity information as a lower limit value. For example, if the similarity information of the gene sequence in the claims is 70%, it means that it should fall within the protection scope of the claims, as long as the similarity to the gene sequence is greater than or equal to 70%. Therefore, the percentage characterized by the similarity information can be taken as the lower limit value of the scope of claims of the gene sequence, thereby determining the protection scope of the gene sequence in the claims.

In practical applications, a certain gene sequence is sometimes directly protected in the claims of the patent application, and the details of the gene sequence can be presented in the claim. Alternatively, the gene sequence may be replaced by a sequence identifier in the claims, and the details of the gene sequence may be presented in the sequence table. In this case, after the descriptive texts of the gene sequence are extracted from the claims and the semantics of the descriptive texts is analyzed by using the trained natural language model, it can be confirmed that what is claimed is the gene sequence itself. That is, the similarity information of the gene sequence determined according to the descriptive texts is an effective percentage of 100%. In this way, for this type of patent applications, after a gene sequence is recognized from the claims, the claimed protection scope of the gene sequence is the gene sequence itself, and the corresponding similarity information is 100%.

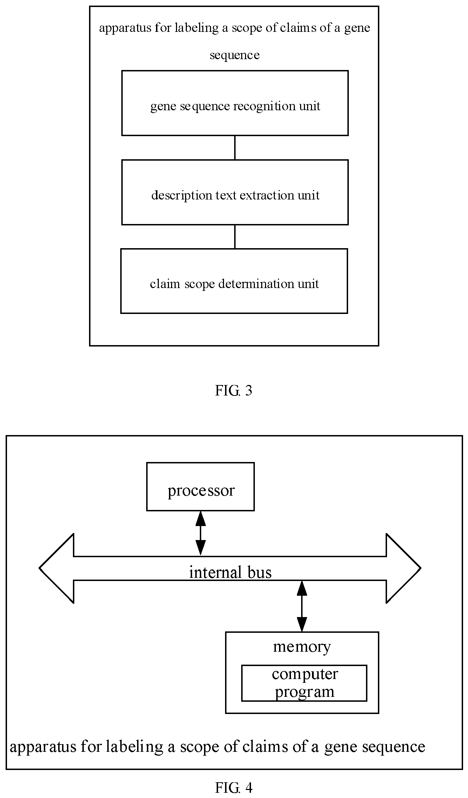

Referring to FIG. 3, there is further provided in the present application a system for labeling a protection scope of claims of a gene sequence, the system comprising:
a gene sequence recognition unit for recognizing a gene sequence from claims of a current patent application;
a descriptive text extraction unit for extracting descriptive texts of the gene sequence from the claims based on a preset keyword; and
a claim scope determination unit for determining similarity information of the gene sequence based on the extracted descriptive texts, and labeling a scope of the claims of the gene sequence based on the similarity information.

Referring to FIG. 4, there is further provided in the present application a system for labeling a protection scope of claims of a gene sequence, which, at the hardware level, may include a processor, an internal bus, and a memory. The memory may include an internal memory and a non-volatile memory. The processor reads the corresponding computer program from the non-volatile memory into the memory and then runs it. The computer program, when being executed by the processor, may implement the above-described method for labeling a protection scope of claims of a gene sequence. It will be understood by those of ordinary skill in the art that the structure illustrated in FIG. 4 is merely illustrative and does not limit the structure of the recognition system described above. For example, the recognition system may further comprise more or fewer components than those illustrated in FIG. 4, for example it may also comprise other processing hardware, such as a GPU (Graphics Processing Unit), or have a configuration different from that illustrated in FIG. 4. Of course, in addition to the software implementation, the present application does not exclude other implementations, such as a logic device or a combination of hardware and software or the like.

It should be noted that the system described above in the embodiments of this disclosure may further comprise other implementations according to the description of the related method embodiments. The specific implementation may refer to the description of the method embodiments, which is not described in detail here.

There is further provided in the present application a computer storage medium for storing a computer program which, when being executed by a processor, can implement the method for labeling a protection scope of claims of a gene sequence as described above.

In the embodiments, the computer storage medium may include a physical system for storing information which generally is digitized and then stored in a medium using an electrical, magnetic or optical method. The computer storage medium described in the present embodiment may further include a system for storing information in an electric energy manner, such as RAM, ROM, and the like; a system for storing information by means of magnetic energy, such as a hard disk, a floppy disk, a magnetic tape, a magnetic core memory, a bubble memory, a U disk; and a system for storing information optically, such as a CD or a DVD. Of course, there are other memories, such as a quantum memory, a graphene memory, and the like.

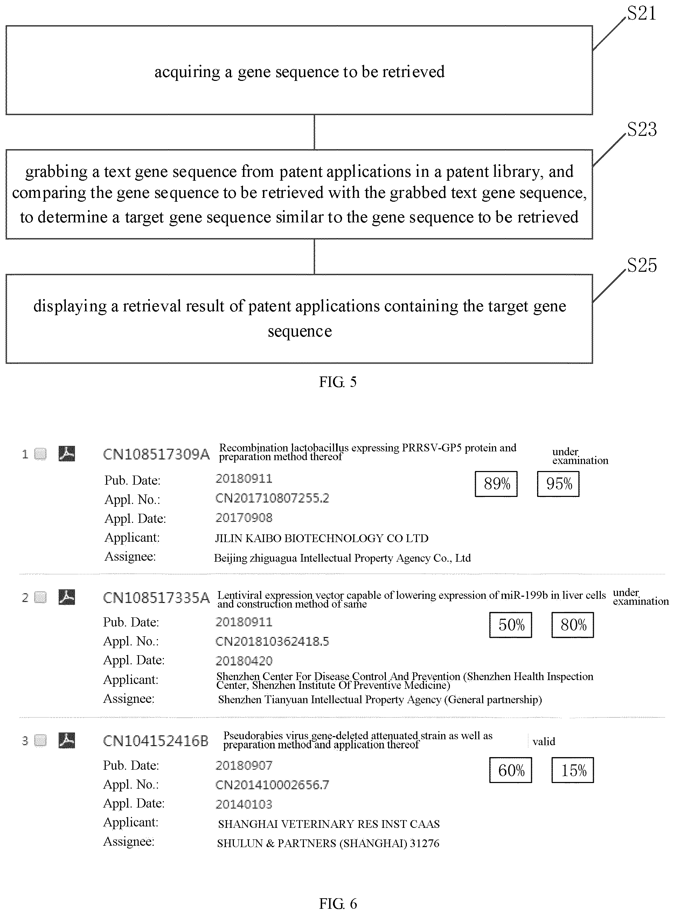

Referring to FIG. 5, there is further provided in the present application a method for retrieving a gene sequence, the method comprising the following steps of:
S21: acquiring a gene sequence to be retrieved;
S23: grabbing a text gene sequence from a patent application in a patent library, and comparing the gene sequence to be retrieved with the grabbed text gene sequence, to determine a target gene sequence similar to the gene sequence to be retrieved; and
S25: displaying a retrieval result of the patent application(s) containing the target gene sequence.

In the embodiments, the gene sequence to be retrieved may be a gene sequence entered by the user in the retrieval engine. For the gene sequence to be retrieved, the retrieval engine can query, from the patent library, patent application(s) related to the gene sequence to be retrieved. Specifically, a text gene sequence can be grabbed from the patent applications in the patent library. The text gene sequence may be a gene sequence contained in the claims or specification of the patent application. When grabbing this text gene sequence, a letter sequence consisted of the specified letters can be recognized from the claims of the current patent application by using the manner shown in FIG. 2, and the letter sequence can be taken as the text gene sequence when the length of this letter sequence reaches a specified length threshold. Moreover, it is also possible to recognize a target sequence identifier from the claims of the current patent application, and to read a text gene sequence corresponding to the target sequence identifier, from the sequence table of the current patent application.

In the embodiments, after the text gene sequence is grabbed, the similarity between the gene sequence to be retrieved and the text gene sequence may be calculated according to a preset algorithm. If the calculated similarity is greater than or equal to a specified similarity threshold, it indicates that the text gene sequence is similar to the gene sequence to be retrieved, and the text gene sequence thus can be regarded as a target gene sequence similar to the gene sequence to be retrieved. In practical application, the preset algorithm may be one or more of algorithms such as KMP, Shift-And, Shift-Or, BM (Boyer-Moore), Horspool, BNDM, BOM, and etc. The specified similarity threshold may be a default value set by the retrieval engine, which may be, for example, a numerical value such as 20% or the like.

In the embodiments, after the target gene sequence similar to the gene sequence to be retrieved is determined, the patent applications containing the target gene sequence can be determined from the patent library, and in this way, the determined patent applications may all be patent applications related to the gene sequence to be retrieved. A retrieval result of these patent applications may be displayed to the user by the retrieval engine, such that the user can further browse the details of each patent application.

In the embodiments, after the target gene sequence similar to the gene sequence to be retrieved is determined, it is also possible to determine a scope of claims of the target gene sequence in a patent application. Specifically, a patent application containing the target gene sequence may be determined at first, and then the descriptive texts of the target gene sequence can be extracted from claims of the patent application according to a preset keyword(s). Specifically, the natural language model may be obtained by training with the NLP technology in the manner of the foregoing embodiments, and then the position where the target gene sequence is located may be recognized in the claims of the patent application by using the natural language model. After the position where the target gene sequence is located is recognized, a length identifier that defines a length of a partial subsequence in the target gene sequence can be looked up from the context of the recognized position by the natural language model. The length identifier can then be the text used to describe the percentage and the regional information of the subsequence as mentioned above. In this way, the descriptive texts of the target gene sequence can be obtained by taking the length identifier as a preset keyword and intercepting the sentence containing the length identifier and the target gene sequence. Finally, similarity information of the target gene sequence can be determined based on the extracted descriptive texts, and the scope of claims of the target gene sequence can be labeled based on the similarity information. Specifically, in one aspect, a percentage of a similarity variable range to which the target gene sequence corresponds may be recognized in the descriptive texts, and the percentage of the similarity variable range is taken as similarity information of the target gene sequence. Herein, the percentage of the similarity variable range may have a starting value and an ending value, and the starting value may be the effective percentage described in the above embodiments, and the ending value may be 100%. In another aspect, regional information for defining a position of a gene subsequence or a core function sequence in the target gene sequence can be recognized in the descriptive texts, and a length of the gene subsequence or the core function sequence is determined according to the regional information. Then, a percentage of the length of the gene subsequence or the core function sequence in the total length of the target gene sequence may be calculated, and the calculated percentage may be taken as the similarity information of the target gene sequence. In the embodiments, after the similarity information of the target gene sequence is determined, the percentage representing the similarity information may be taken as a lower limit value of the scope of claims of the target gene sequence. In this way, after the retrieval result of the patent applications is provided to the user based on the similarity between the gene sequences, the scope of claims of the target gene sequence can also be displayed in the retrieval result. The scope of claims can then be expressed by a percentage numerical value characterizing the lower limit value. In practical application, the retrieval result of the patent applications may be presented to the user as shown in FIG. 6. The retrieval result may include common information of the patent application, such as the title, the publication date, the application number, the applicant, and the agency and the like, and may also include similarity between the gene sequence to be retrieved and the target gene sequence, as well as an identifier for characterizing the scope of claims of the target gene sequence. Herein, the similarity and the identifier for characterizing the scope of claims may both be expressed in a form of a percentage. For example, in FIG. 6, in the first retrieval result, 89% represents the similarity between a target gene sequence in that patent application and the gene sequence to be retrieved, and 95% represents a lower limit value of the scope of claims of the target gene sequence. In practical application, the retrieval engine can sort the retrieval results according to the rules set in the background, the similarity, or the scope of claims.

In the embodiments, when the user inputs a gene sequence to be retrieved, the user may also define a specified similarity range corresponding to the gene sequence to be retrieved. The specified similarity range can filter the retrieval results obtained according to the similarity query. Specifically, the retrieval engine may select, from the target gene sequence, a selected gene sequence whose similarity to the gene sequence to be retrieved is within the specified similarity range, and display a retrieval result of the patent applications containing the selected gene sequence. In this way, the similarity between the target gene sequence contained in the patent applications and the gene sequence to be retrieved finally obtained by query may all be within the specified similarity range. Therefore, the retrieval result can be made more in line with the user's expectation, and the accuracy of the retrieval result can be improved.

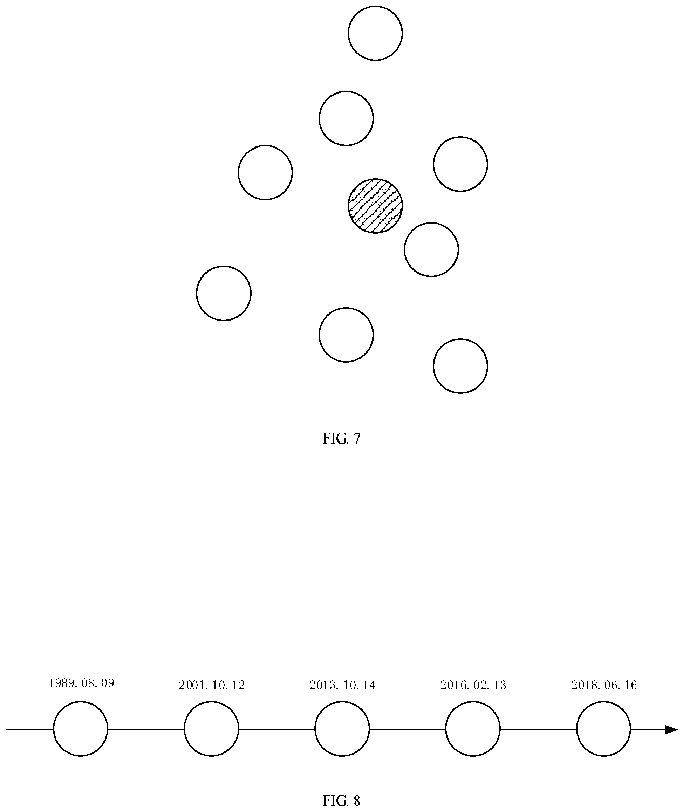

In the embodiments, in order to more intuitively present the retrieval result to the user, after the patent applications matching the gene sequence to be retrieved are queried, the patent applications obtained by query may be converted into a similarity map according to the similarity between the target gene sequence in the patent applications and the gene sequence to be retrieved. Referring to FIG. 7, in the similarity map, the gene sequence to be retrieved may be located at the center of the map (a circle filled with shadow lines), the patent applications matching the gene sequence to be retrieved may be distributed all around, and a distance between a patent application and the gene sequence to be retrieved can be determined by the similarity between the target gene sequence in the patent application and the gene sequence to be retrieved. The higher the similarity is, the closer the distance is. Thus, in the similarity map, the distance between the patent application and the gene sequence to be retrieved is inversely proportional to the similarity. The user can see intuitively the distribution of the retrieval result through a visual display mode of the similarity map, and after a patent application in the similarity map is clicked, it is possible to jump to the detailed page of the corresponding patent application for the user to further browse.

In the embodiments, for the displayed similarity map, the user can further adjust the similarity range, so that the result displayed in the similarity map changes accordingly. Specifically, the retrieval engine may receive a similarity range input by the user and remove the patent application having a similarity outside the similarity range from the similarity map. In this way, the user can input a similarity range concerned by himself, so that the patent applications satisfying the similarity range in the similarity map can be retained, so as to reduce the influence of other patent applications.

In practical application, after the user inputs the similarity range, the retrieval engine can still retain all the retrieval results, but the retrieval results within and outside the similarity range are displayed with different colors, so as to distinguish such different patent applications to the user. Specifically, the retrieval engine may receive a similarity range input by the user, determine in the similarity map a first patent application having a similarity within the similarity range, determine in the similarity map a second patent application having a similarity outside the similarity range, and then display the first patent application and the second patent application in different colors in the similarity map. For example, the second patent application, which is outside the similarity range, may be displayed in gray, while the first patent application within the similarity range may be display in red.

In the embodiments, there may also be other aspects of the user's need for the retrieval result of the gene sequence, for example, the user sometimes wants to learn the development history of a gene sequence. In this case, the retrieval engine may provide the user with a function of displaying the development history. When the user triggers this function, the retrieval engine can construct the patent applications obtained by query into a gene sequence development progress map according to the information of filing date. Specifically, referring to FIG. 8, the retrieved patent applications may form an application sequence in which the order from left to right corresponds to application dates from old to recent. In this way, the user can clearly see the progress of the development of the gene sequence.

Furthermore, in the embodiments, the user may be interested only in the core function sequence in the gene sequence to be retrieved, and want to learn the development history of the core functional sequence. The core function sequence may be a partial gene sequence which is important in the gene sequence. For example, among many gene sequences in a human body, a portion of the nucleotide sequences can characterize a potential cancer risk in the body. In this case, these nucleotide sequences can be used as the core function sequence in the gene sequences. In practical application, a sequence library of individual core function sequences can be constructed in advance in the patent library, so that after receiving the gene sequence to be retrieved input by a user, the retrieval engine may compare the gene sequence to be retrieved with the core function sequences in the sequence library, so as to recognize the contained core function sequence from the gene sequence to be retrieved. The retrieval engine may then select the target patent applications containing the core function sequence from the patent applications obtained by query. In this way, the retrieval engine can construct the target patent applications as a core sequence development progress map according to the information of filing date. Similarly, the target patent applications may form an application sequence in which the order from left to right corresponds to application dates from old to recent. In this way, the user can clearly see the development progress of the core function sequence in the gene sequence.

There is further provided in the present application a system for retrieving a gene sequence, the system comprising:
a retrieval information acquisition unit for acquiring a gene sequence to be retrieved;
a target gene sequence retrieval unit for grabbing a text gene sequence from a patent application of a patent library, and comparing the gene sequence to be retrieved with the grabbed text gene sequence, to determine a target gene sequence similar to the gene sequence to be retrieved; and
a retrieval result displaying unit for displaying a retrieval result of patent application(s) containing the target gene sequence.

There is further provided in the present application a system for retrieving a gene sequence, the system comprising: a processor and a memory for storing a computer program which, when being executed by the processor, can implement the method for retrieving a gene sequence as described above.

There is further provided in the present application a computer storage medium for storing a computer program which, when being executed by a processor, can implement the method for retrieving a gene sequence as described above.

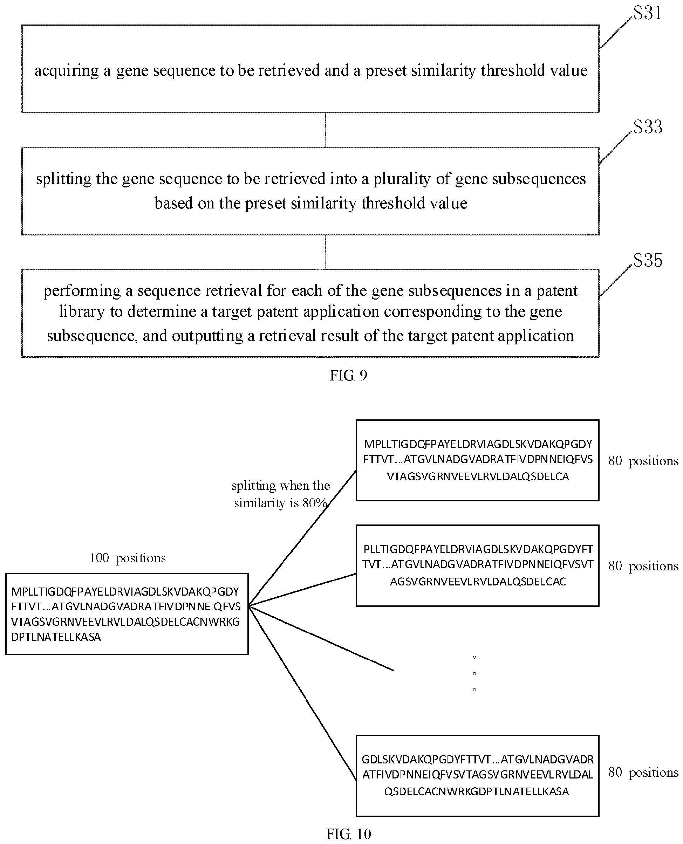

Referring to FIG. 9, there is further provided in the present application a method for retrieving a gene sequence, the method may comprise the following steps.

S31: acquiring a gene sequence to be retrieved and a preset similarity threshold value.

S33: splitting the gene sequence to be retrieved into a plurality of gene subsequence based on the preset similarity threshold value.

In the embodiments, the user can input the gene sequence to be retrieved along with the preset similarity threshold to be retrieved. The preset similarity threshold may be, for example, 80%, which indicates that the user wants to query a patent application in which the similarity to the gene sequence to be retrieved is 80% or higher. In this case, on one hand, the retrieval engine can query similar patent applications in the patent library according to the gene sequence to be retrieved. Then, the patent applications obtained by query are filtered according to the preset similarity threshold. In another aspect, the gene sequence to be retrieved can be split according to the preset similarity threshold value.

Referring to FIG. 10, when the gene sequence to be retrieved is split, a target length of the split gene subsequence may be determined according to the preset similarity threshold value and a length of the gene sequence to be retrieved. For example, when the length of the gene sequence to be retrieved is 100 and the preset similarity threshold value is 80%, the target length of the split gene subsequence may be 80. At this time, the subsequence(s) conforming to the target length may be extracted from the gene sequence to be retrieved, and the extracted subsequence(s) may be used as the split gene subsequence(s). For example, for a gene sequence to be retrieved which has a length of 100, at least 21 different gene subsequences having a length of 80 may be extracted therefrom. These extracted gene subsequence(s) can be used as the split gene subsequence(s).

S35: performing a sequence retrieval for each of the gene subsequences in a patent library, to determine target patent application(s) corresponding to the gene subsequences, and outputting a retrieval result of the target patent application(s).

In the embodiments, for different gene subsequences, a sequence retrieval can be performed separately in the patent library. The retrieval aims to query the patent application(s) containing the gene subsequence. This can ensure that the similarity between the gene sequence in the retrieved patent application and the gene sequence to be retrieved is greater than or equal to 80%, thereby satisfying the preset similarity threshold value input by the user.

In the embodiments, the method shown in FIG. 5 can be referred to when a sequence retrieval is performed for a gene subsequence. Firstly, a text gene sequence may be grabbed from a patent application of a patent library, and whether the grabbed text gene sequence contains the gene subsequence may be determined. If the grabbed text gene sequence contains the gene subsequence, the patent application containing the text gene sequence may be regarded as a target patent application corresponding to the gene subsequence. The target patent application can be regarded as a retrieval result for the gene sequence to be retrieved.

In the embodiments, when a text gene sequence is grabbed from a patent application in a patent library, the text gene sequence may be grabbed from the claims and the specification, respectively. To be specific, it is possible, on one hand, to recognize a letter sequence consisted of specified letters from the claims of the current patent application, and to take the letter sequence as the text gene sequence when the length of the letter sequence reaches a specified length threshold; and on the other hand, to recognize a target sequence identifier from the claims of the current patent application, and to read a text gene sequence corresponding to the target sequence identifier, from the sequence table of the current patent application.

There is further provided in the present application a system for retrieving a gene sequence, the system comprising:
a retrieval information acquisition unit for acquiring a gene sequence to be retrieved and a preset similarity threshold value;
a subsequence splitting unit for splitting the gene sequence to be retrieved into a plurality of gene subsequence based on the preset similarity threshold value; and
a retrieval result outputting unit for performing a sequence retrieval for each of the gene subsequences in a patent library, to determine a target patent application corresponding to the gene subsequence, and outputting a retrieval result of the target patent application.

There is further provided in the present application a system for retrieving a gene sequence, the system comprising: a processor and a memory for storing a computer program which, when being executed by the processor, implements the method for retrieving a gene sequence as described above.

There is further provided in the present application a computer storage medium for storing a computer program which, when being executed by a processor, implements the method for retrieving a gene sequence as described above.

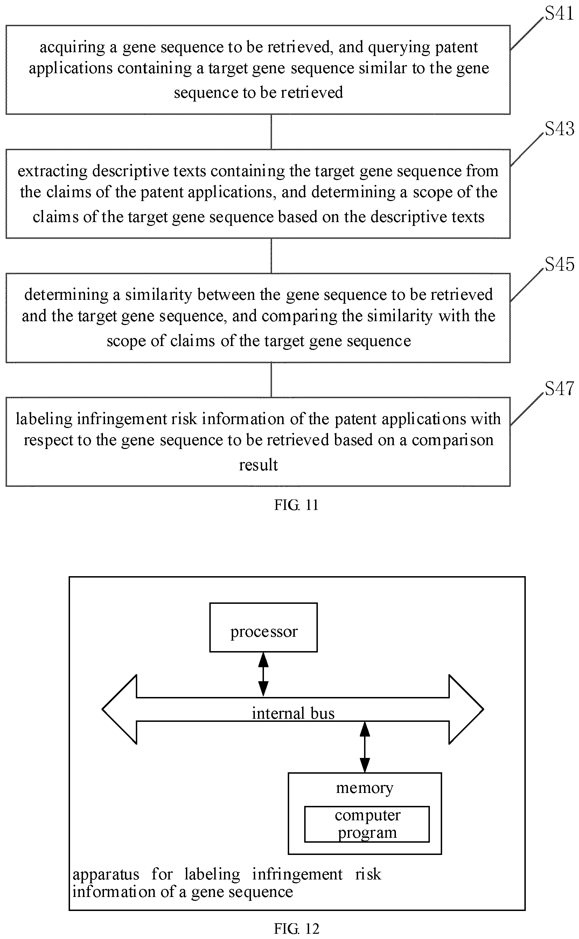

There is provided in the present application a method for labeling infringement risk information of a gene sequence, which can be applied to a retrieval engine. Referring to FIG. 11, the method may comprise the following steps.

S41: acquiring a gene sequence to be retrieved, and querying the patent application(s) containing a target gene sequence similar to the gene sequence to be retrieved.

In the embodiments, the gene sequence to be retrieved may be a string of letters describing DNA, RNA, other nucleotides or proteins. In the gene sequence to be retrieved, each letter may be used as an abbreviated character for nucleotides or amino acids. In this way, DNA, RNA, other nucleotides or proteins having a complex biological structure can be converted into a corresponding gene sequence in the form of characters.

In the embodiments, when a user wants to retrieve a gene sequence, he can input the gene sequence to be retrieved into a keyword input box of the retrieval engine, and in this way the retrieval engine can acquire the gene sequence to be retrieved. The retrieval engine may, by means of a text search, query a patent application matching the gene sequence to be retrieved in a retrieval library. Specifically, in a patent application containing a gene sequence, the gene sequence can be represented by means of a sequence table. In this way, the retrieval engine can perform a text-matching between the gene sequence to be retrieved and each gene sequence contained in the sequence table of the patent application, so as to query a target gene sequence similar to the gene sequence to be retrieved. Herein, the association between the gene sequence to be retrieved and the target gene sequence can be expressed by the similarity therebetween. The higher the similarity is, the closer the association between the two is. When the similarity between the gene sequence to be retrieved and the target gene sequence is calculated, letters at the same positions in the two gene sequences may be compared one by one, and the same letters may be labeled. Thus, after the two gene sequences are compared, a proportion of the same letters in the gene sequence to be retrieved can be calculated, and this proportion can be used as the similarity between the two gene sequences. It should be noted that, in a single patent application, several target gene sequences similar to the gene sequence to be retrieved may be provided, then the retrieval engine may calculate the similarities between the target gene sequences and the gene sequence to be retrieved, respectively, and save the calculated similarities in the background.

Of course, in practical application, sometimes the gene sequences appearing in the patent application are not all protected in the claims, and some gene sequences may be exemplified in the specification for the purpose of illustrating the technical solution. In this case, since it is subsequently necessary to determine the protection scope of the target gene sequences, those target gene sequences that do not appear in the claims can be discarded when similarity retrieval is performed. To be specific, for patent applications in a retrieval library, the contained gene sequences may firstly be recognized from the claims, the similarities between these gene sequences contained in the claims and the gene sequence to be retrieved are then calculated. In this way, it is ensured that the finally determined target gene sequences are all presented in the claims.

In the embodiments, if the number of patent applications queried according to the similarity between gene sequences is large, the retrieval engine can filter the patent applications obtained by query according to similarity in the background, thereby retaining the patent applications having a high similarity.

S43: extracting descriptive texts containing the target gene sequence from the claims of the patent application, and determining a scope of claims of the target gene sequence based on the descriptive texts.

In the embodiments, after the patent application is obtained by query according to the similarity of the gene sequences, the scope of claims of the target gene sequence defined in the patent application can be further determined. Specifically, the claims of the patent application may be analyzed to extract descriptive texts containing the target gene sequence from the claims, and the scope of claims of the target gene sequence may be determined based on the descriptive texts.

In the embodiments, the scope of claims of the target gene sequence defined in the claims can be recognized by performing a semantic analysis of the claims of the patent application by using a natural language processing (NLP) technique. Specifically, the retrieval engine may take a large number of claims describing gene sequences as training samples in advance, and train a natural language model by using machine learning, which can recognize the meanings characterized by different phrases in the claims and the associations among multiple phrases. During the training of the natural language model, firstly, text related to description of the protection scope of the gene sequence can be labeled in the training samples through manual labeling. For example, for the following training samples:

"A polypeptide structure comprising at least 70% of the amino acids in SEQ ID No. 2, or amino acids at positions 48 to 56, 90 to 101, and 161 to 169 in SEQ ID No. 4."

In these training samples, the cited gene sequences can be labeled. For example, the "SEQ ID No. 2" and "SEQ ID No. 4" in the above example can be labeled with seq_id. Moreover, the text used to define the length of a partial subsequence in the gene sequence may also be labeled. Herein, the text describing the length of the subsequence may include a percentage numerical value, such as "70%" as described above, and may also include regional information for defining the position of a subsequence in the gene sequence, such as "48 to 56," "90 to 101 " and "161 to 169 ", as described above. When the text describing the length of a subsequence is labeled, it can be labeled differently according to different types. For example, for a percentage numerical value, it can be labeled with "quantity"; and for regional information, it can be labeled with "region". Moreover, the text describing the length of the subsequence will typically be associated with the cited gene sequence, for example, "70%" in the above example is associated with SEQ ID No. 2, and the texts "48 to 56," "90 to 101" and "161 to 169" are associated with the SEQ ID No. 4. Therefore, when the manual labeling is performed, two objects that are associated with each other can also be labeled in a form of key-value. For example, the labeling result may be "70% - SEQ ID No. 2", "48 to 56, 90 to 101, 161 to 169 - SEQ ID No. 4". Of course, in practical application, the above-mentioned labeling manner can also be changed flexibly according to different rules, and the above exemplary labeling situation is only for describing the technical solution of this embodiment, which is not meant that the technical solution of the embodiments can be labeled only by the above labeling method. In this way, the labeled training samples can be input into the natural language model, and a neural network in the natural language model can learn the labeled learning samples, so as to distinguish the semantics of each sentence and the correlation between different sentences according to the way of presenting the claims. After training a large number of samples, the trained natural language model can recognize the claims that are not labeled, so as to recognize the gene sequence and the text describing the length of the subsequence therefrom, which are labeled at the training stage.

In the embodiments, a trained natural language model may be integrated into the retrieval engine, then after the patent application is obtained by query according to the similarity, a sequence identifier of the target gene sequence in the claims may firstly be determined from the sequence table of the patent application, and the position where the sequence identifier is located may be recognized in the claims by using the natural language module. After the position where the sequence identifier is located is recognized, a length identifier that defines the length of a partial subsequence in the target gene sequence can be looked up from the context of the recognized position by a natural language model. The length identifier can then be the text used to describe the percentage and the regional information of the subsequence as mentioned above. In this way, the descriptive texts containing the target gene sequence can be obtained by intercepting the sentence containing the length identifier and the sequence identifier.

In the embodiments, after the descriptive texts containing the target gene sequence are obtained by extraction, semantic analysis may be performed on the descriptive texts, so as to determine the scope of claims of the target gene sequence in the descriptive texts. Specifically, if the extracted descriptive texts contain a percentage numerical value for describing the target gene sequence, a percentage of a similarity variable range to which the target gene sequence corresponds may be recognized directly in the descriptive texts, and the percentage of the similarity variable range is taken as the scope of claims of the target gene sequence. Herein, the percentage of the similarity variable range may have a starting value and an ending value, and the starting value may be the effective percentage described in the above embodiments, and the ending value may be 100%. For example, in the above example, "70%" can be used as the lower limit value of the similarity variable range. However, in some patent applications, a percentage numerical value of the target gene sequence is not described directly, but the regional information of the subsequence therein is described, such as "48 to 56", "90 to 101" and "161 to 169" described in the above example for the SEQ ID No. 4. In this case, regional information for defining a position of a gene subsequence or a core function sequence in the target gene sequence can be recognized in the descriptive texts, and a length of the gene subsequence or the core function sequence is determined according to the regional information. In the above example, the gene subsequence or the core function sequence is composed of three parts, the sum of lengths of which is 9 + 12 + 9 = 30. Then, a percentage of the length of the gene subsequence or the core function sequence in the total length of the gene sequence may be calculated, and the calculated percentage may be taken as the lower limit value of the scope of claims of the target gene sequence. Assuming that the total length of SEQ ID No. 4 is 200, the calculated percentage is 30/200 = 15%, and therefore, the lower limit value of the scope of claims of SEQ ID No. 4 is 15%.

S45: determining a similarity between the gene sequence to be retrieved and the target gene sequence, and comparing the similarity with the scope of claims of the target gene sequence.

S47: labeling infringement risk information of the patent application with respect to the gene sequence to be retrieved based on a comparison result.

In the embodiments, the similarity between a gene sequence to be retrieved and a target gene sequence can be determined at the time of similarity retrieval. According to the description in the step S43, it is also possible to determine the scope of claims of the target gene sequence in the patent application. At that time, on one hand, the retrieval result of the patent application(s) may be presented to the user as shown in FIG. 6. The retrieval result may include common information of the patent application, such as the title, the publication date, the application number, the applicant, and the agency and the like, and may also include the similarity between the gene sequence to be retrieved and the target gene sequence as well as an identifier for characterizing the protection scope of the target gene sequence. Herein, the similarity and the identifier for characterizing the protection scope may both be expressed in a form of a percentage. For example, in FIG. 6, in the first retrieval result, 89% represents the similarity between the target gene sequence in the patent application and the gene sequence to be retrieved, and 95% represents the protection scope of the target gene sequence. In practical application, the retrieval engine can sort the retrieval results according to the rules set in the background, the similarity, or the protection scope.

It should be noted that there may be at least two target gene sequences similar to the gene sequence to be retrieved in some of the retrieved patent applications. In this case, the similarity and the protection scope of each target gene sequence can be determined separately, and then the similarities and the protection scopes of these target gene sequences can be listed in the retrieval result for query by the user.

In practical application, the similarity and the protection scope displayed in the retrieval result can be displayed by means of hyperlinks. When the user clicks on the similarity or the protection scope therein, it is possible to directly jump to the details of the patent application, and to display the content related to the similarity or the protection scope on the current page. For example, if the user clicks the protection scope in the first retrieval result, the claims part of the first retrieval result can be directly displayed on the jumped page, and the text used to represent the protection scope can be displayed by means of highlighting.

On the other hand, the retrieval engine may compare the similarity and the scope of claims in the background, and if the similarity falls within the scope of claims, it indicates that the current gene sequence to be retrieved is at risk of infringement. For example, a similarity between a gene sequence to be retrieved and the target gene sequence is 80%, and the scope of claims of the target gene sequence is 75% or higher. Then the gene sequence to be retrieved is within the protection scope of the target gene sequence, at this time, the retrieval engine may label the infringement risk information of the patent application with respect to the gene sequence to be retrieved in the retrieval result. In practical application, the infringement risk information can be represented by a combination of text and background color. For example, when the gene sequence to be retrieved is within the protection scope of the target gene sequence, the word "infringement" may be labeled, and the background color of the word "infringement" can be red. For another example, when the gene sequence to be retrieved is outside the protection scope of the target gene sequence, but a difference between the similarity and the lower limit value of the scope of claims is small, the words "possible infringement" may be labeled, and the background color of the words may be yellow. For another example, when the gene sequence to be retrieved is outside the protection scope of the target gene sequence, but a difference between the similarity and the lower limit value of the scope of claims is large, the words "no infringement risk" may be labeled, and the background color of the words may be green. Of course, in practical application, the infringement risk information may also be labeled by other means, which is not limited thereto herein.

In the embodiments, when the user inputs the gene sequence to be retrieved, the user may also define a specified similarity range corresponding to the gene sequence to be retrieved. The specified similarity range can filter the retrieval results obtained according to the similarity query. Specifically, the retrieval engine may receive the specified similarity range input by the user, and query according to the similarity to obtain the patent applications corresponding to the gene sequence to be retrieved, and thereafter the similarity of the target gene sequence in each of the patent applications can be calculated. Then, the calculated similarity may be compared with the specified similarity range input by the user, so that the retrieval result within the specified similarity range is retained. In this way, the similarity between the target gene sequence contained in the patent application and the gene sequence to be retrieved finally obtained by query may all be within the specified similarity range, therefore, the retrieval result can be made more in line with the user's expectation, and the accuracy of the retrieval result can be improved.

In the embodiments, in order to more intuitively present the retrieval result to the user, after the patent application(s) matching the gene sequence to be retrieved is queried, the patent application(s) obtained by query may be converted into a similarity map according to the similarity between the target gene sequence in the patent application and the gene sequence to be retrieved. Referring to FIG. 7, in the similarity map, the gene sequence to be retrieved may be located at the center of the map (a circle filled with shadow lines), and the patent application matching the gene sequence to be retrieved may be distributed all around, and a distance between the patent application and the gene sequence to be retrieved can be determined by the similarity between the target gene sequence and the gene sequence to be retrieved in a patent application. The higher the similarity is, the closer the distance is. Thus, in the similarity map, the distance between the patent application and the gene sequence to be retrieved is inversely proportional to the similarity. The user can see intuitively the distribution of the retrieval result through a visual display mode of the similarity map, and after the patent application in the similarity map is clicked, it is possible to jump to the detailed page of the corresponding patent application for further browsing by the user.

In the embodiments, for the displayed similarity map, the user can further adjust the similarity range so that the result displayed in the similarity map changes accordingly. Specifically, the retrieval engine may receive a similarity range input by the user and remove a patent application having a similarity outside the similarity range from the similarity map. In this way, the user can input the similarity range concerned by himself, so that the patent applications satisfying the similarity range in the similarity map can be retained, so as to reduce the influence of other patent applications.

In practical application, after the user inputs the similarity range, the retrieval engine can still retain all the retrieval results, but the retrieval results within and outside the similarity range are displayed by different colors, so as to distinguish such different patent applications to the user. Specifically, the retrieval engine may receive a similarity range input by the user, determine in the similarity map a first patent application having a similarity within the similarity range, determine in the similarity map a second patent application having a similarity outside the similarity range, and display the first patent application and the second patent application in different colors in the similarity map. For example, the second patent application, which is outside the similarity range, may be displayed in gray, while the first patent application within the similarity range may be displayed in red.

In the embodiments, there may also be other aspects of the user's need for the retrieval result of the gene sequence, for example, the user sometimes wants to learn the development history of the gene sequence. In this case, the retrieval engine may provide the user with a function of displaying the development history. When the user triggers the function, the retrieval engine can construct the patent application obtained by query into a gene sequence development progress map according to the information of filing date. Specifically, referring to FIG. 8, the retrieved patent applications may constitute an application sequence in which the order from left to right corresponds to application dates from old to recent. In this way, the user can clearly see the progress of the development of the gene sequence.

Furthermore, in the embodiments, the user may be interested only in the core function sequence in the gene sequence to be retrieved, and want to learn the development history of the core functional sequence. The core function sequence may be a partial gene sequence which is important in the gene sequence. For example, among many gene sequences of a human body, a portion of the nucleotide sequences can characterize a potential cancer risk in the body. In this case, these nucleotide sequences can be used as the core function sequence in the gene sequences. In practical application, a sequence library of individual core function sequences can be constructed in advance in the patent library, so that after receiving the gene sequence to be retrieved input by a user, the retrieval engine may compare the gene sequence to be retrieved with the core function sequences in the sequence library, so as to recognize the contained core function sequence from the gene sequence to be retrieved. The retrieval engine may then filter the target patent application containing the core function sequence from the patent applications obtained by query. In this way, the retrieval engine can construct the target patent application as a core sequence development progress map according to the information of filing date. Similarly, the target patent application may form an application sequence in which the order from left to right corresponds to application dates from old to recent. In this way, the user can clearly see the development progress of the core function sequence in the gene sequence.

There is further provided in the present application a system for labeling infringement risk information of a gene sequence, the system comprising:
a patent application querying unit for acquiring a gene sequence to be retrieved, and querying the patent application(s) containing a target gene sequence similar to the gene sequence to be retrieved;
a claim scope determination unit for extracting descriptive texts containing the target gene sequence from the claims of the patent application, and determining a scope of claims of the target gene sequence based on the descriptive text;
a comparison unit for determining a similarity between the gene sequence to be retrieved and the target gene sequence, and comparing the similarity with the scope of claims of the target gene sequence; and
a risk judgment unit for labeling infringement risk information of the patent application with respect to the gene sequence to be retrieved, based on a comparison result.

There is further provided in the embodiments of the present disclosure a system for labeling infringement risk information of a gene sequence, the system comprising: a processor and a memory for storing a computer program which, when being executed by the processor, implements the method for labeling infringement risk information of a gene sequence as described above. Specifically, as shown in FIG. 12, at the hardware level, the system may include a processor, an internal bus, and a memory. The memory may include an internal memory and a non-volatile memory. The processor reads the corresponding computer program from the non-volatile memory into the memory and then runs it. It will be understood by those of ordinary skill in the art that the structure shown in FIG. 12 is merely illustrative and does not intend to limit the structure of the recognition system described above. For example, the recognition system may further comprise more or fewer components than those shown in FIG. 12, for example it may also comprise other processing hardware, such as a GPU (Graphics Processing Unit), or have a configuration different from that shown in FIG. 12. Of course, in addition to the software implementation, the present application does not exclude other implementations, such as a logic device or a combination of hardware and software or the like.

In the embodiments of the present disclosure, the processor may include a central processing unit (CPU) or a graphics processing unit (GPU), and of course, it may also include other single-chip computers with logic processing capability, logic gate circuits, integrated circuits, and etc., or appropriate combinations thereof. The memory described in the embodiments of the present application may be a memory device for storing information. In the digital system, a device capable of storing binary data may be a memory; in the integrated circuit, a circuit that has no physical form but has a storage function may also be a memory, such as a RAM, a FIFO, and etc.; and in the system, a storage device that has a physical form may also be referred to as a memory, etc. In implementation, the memory may also be implemented in the form of a cloud memory, and the specific implementation is not limited in this disclosure.

It should be noted that the system described above in the embodiments of this disclosure may further include other implementations according to the description of the related method embodiments. The specific implementation may refer to the description of the method embodiments, which is not described in detail herein.

There is further provided in the present application a computer storage medium for storing a computer program which, when being executed by a processor, implements method for labeling infringement risk information of a gene sequence as described above.

As can be seen from the above, in one or more embodiments of the present disclosure, after a gene sequence to be retrieved provided by a user is acquired, a patent application(s) matching the gene sequence to be retrieved can be queried at first in accordance with a similarity between the gene sequences. Specifically, it is possible to grab a text gene sequence from a patent application of a patent library, and to compare the gene sequence to be retrieved with the grabbed text gene sequence, to determine a target gene sequence similar to the gene sequence to be retrieved. The patent application containing the target gene sequence can then be regarded as a patent application which matches the gene sequence to be retrieved. A target gene sequence similar to the gene sequence to be retrieved may be included in these patent applications. However, in scenarios such as infringement retrieval analysis, it is not sufficient to provide only a retrieval result of similarity of the gene sequences. Assuming that the user conducts an infringement retrieval analysis on a gene sequence, the patent application retrieved based on the similarity may have only a target gene sequence with a similarity of 90%, but have no completely consistent gene sequence. However, it can not be concluded that the gene sequence can be used freely at this time. The reason is that the target gene sequence contained in the retrieved patent application usually has a protection scope, this protection scope may cover, for example, a gene sequence having a similarity of 80% or more to the target gene sequence involved in the patent application. Thus, since the similarity between the gene sequence to be retrieved and the target gene sequence is 90%, which actually falls within the protection scope of the patent application, and thus there is still a possibility of infringement. In view of this, in the present disclosure, after the patent application is obtained by performing retrieval based on the similarity, descriptive texts containing the target gene sequence may be further extracted from the claims of the patent application, and a protection scope of the target gene sequence may be determined according to the descriptive texts. Then, the similarity corresponding to the patent application along with the protection scope can be displayed in the retrieval result, so that the user can compare the size relationship between the similarity and protection scope, so as to judge whether the gene sequence to be retrieved has the possibility of infringement. As can be seen from the above, the technical solution provided by one or more embodiments of the present disclosure can not only retrieve a gene sequence in the patent library, but also provide users with more abundant retrieval information, thereby improving the accuracy of the retrieval result.

Specific embodiments of the present disclosure have been described in the above. Other embodiments fall within the scope of the appended claims. In some cases, the actions or steps recited in the claims may be performed in a different order from that in the embodiments and still achieve the desired results. In addition, the processes depicted in the drawings do not necessarily require a particular order or a sequential order shown in order to achieve the desired results. In some embodiments, multi-tasking and parallel processing may also be possible or may be advantageous.

Although the present application provides the method operation steps as described in the embodiments or the flowcharts, more or less operation steps may be included based on the conventional or non-creative effort. The order of the steps listed in the embodiments is merely one of various execution orders of the steps, rather than a unique execution order. When being executed at an actual system or client product, the steps may be performed in sequence or in parallel according to the methods illustrated in the embodiments or drawings (e.g., by a parallel processor or under a multi-threaded processing environment).

In the 1990s, an improvement to a technology could be clearly distinguished as a hardware improvement (e.g., an improvement to a circuit structure such as diode, transistor, switch, etc.) or a software improvement (an improvement to a methodical flow). However, with the development of the technology, the improvements to many methodical flows nowadays can be deemed as direct improvements to the hardware circuit structure. The designers almost always obtain the corresponding hardware circuit structure by programming the improved methodical flows into the hardware circuit. Thus, it cannot be said that an improvement to a methodical flow cannot be implemented by a hardware entity module. For example, a Programmable Logic Device (PLD) (such as a Field Programmable Gate Array (FPGA)) is such an integrated circuit whose logic function is determined by the user's programming of the device. The designers themselves program to "integrate" a digital system to a single PLD, without requiring any chip manufacturer to design and manufacture the dedicated integrated circuit chips. Moreover, today, instead of manually making the integrated circuit chips, the programming is mostly implemented using the software "logic compiler". It is similar to the software compiler used in program development and drafting, and the previous original codes also need to be compiled in a specific programming language, which is referred to as the Hardware Description Language (HDL). In addition, there are many types of HDLs, such as Advanced Boolean Expression Language (ABEL), Altera Hardware Description Language (AHDL), Confluence, Cornell University Programming Language (CUPL), HDCal, Java Hardware Description Language (JHDL), Lava, Lola, MyHDL, PALASM, Ruby Hardware Description Language (RHDL), etc. Currently, the Very-High-Speed Integrated Circuit Hardware Description Language (VHDL) and Verilog are most commonly used. It is also be apparent to those skilled in the art the hardware circuit for implementing the logic methodical flows can be easily obtained by slightly programming the methodical flows into the integrated circuit with the above hardware description languages.

Specifically, any system, module or unit set forth in the above embodiments may be implemented by a computer chip or an entity, or by a product having a certain function. A typical implementation device is a computer. To be specific, the computer may be, for example, a personal computer, a laptop computer, a cellular phone, a camera phone, a smart phone, a personal digital assistant, a media player, a navigation device, an email device, a game console, a tablet computer, a wearable device, or combination of any of these devices.

For the convenience of description, when the above mentioned system is to be described, it is divided into various units based on its functions and described separately. Of course, the functions of the various units may be implemented in the same one or more software and/or hardware when the present disclosure is implemented.

Persons skilled in the art shall understand that, the embodiments of the present invention can be provided as a method, a system or a computer program product. Therefore, the present invention can adopt the forms of a complete hardware example, a complete software example, or combination of a software example and a hardware example. Moreover, the present invention can adopt the form of a computer program product that is implemented on one or more computer-usable storage medium (including but not limited to a disk memory, a CD-ROM, an optical memory, and etc.) including computer-usable program codes.

The invention is described with reference to flow diagrams and/or block diagrams of the method, the device (system) and the computer program product according to the embodiment of the invention. It should be understood that each flow and/or block in the flow diagrams and/or block diagrams, and the combination of the flows and/or blocks in the flow diagrams and/or block diagrams can be achieved by computer program commands. These computer program commands can be provided to a CPU of a general-purpose computer, a special-purpose computer, an embedded processor or other programmable data processing device to produce a machine, so that an apparatus for achieving functions specified in one or more flows in the flow diagrams and/or one or more blocks in the block diagrams can be generated by the command executed by the CPU of the computer or other programmable data processing device.

These computer program instructions can also be stored in a computer-readable memory that can guide a computer or other programmable data processing device to operate in a special way, so that the instruction stored in the computer-readable memory generates a manufactured product including an instruction system which achieves functions specified in one or more flows in the flow diagrams and/or one or more blocks in the block diagrams.

These computer program instructions can also be loaded on a computer or other programmable data processing device, on which a series of operation steps are executed to generate processing achieved by the computer, so that the instruction executed on the computer or other programmable data processing device is provided for being used in the steps of achieving functions specified in one or more flows in the flow diagrams and/or one or more blocks in the block diagrams.

In a typical configuration, a computing device includes one or more processors (CPUs), input/output interfaces, network interfaces, and a memory.

The memory, which may have the form of a volatile memory, a Random-Access Memory (RAM) and/or a nonvolatile memory such as Read-Only Memory (ROM) or a flash RAM, etc. among the computer readable medium. The memory is an example of the computer readable medium.

The computer-readable medium includes permanent and non-permanent, removable and non-removable media, which can implement the information storage in any method or technique. The information can be computer readable instructions, data structures, program modules or other data. An example of the computer storage medium includes, but not limited to, a phase change memory (PRAM), a static random access memory (SRAM), a dynamic random access memory (DRAM), other types of random access memory (RAM), a read-only memory (ROM), an electrically-erasable programmable read-only memory (EEPROM), a flash memory or other memory techniques, a compact disk read only memory (CD-ROM), a digital versatile disc (DVD) or other optical storages, magnetic cassette tapes, magnetic diskettes, graphene storage or other magnetic storage device, or any other non-transmission medium, which can be used for the storage of information accessible to a computing device. According to the definitions herein, the computer readable medium does not include any temporary computer readable media (transitory media), such as modulated data signal and carrier wave.

It is also to be understood that the terms "comprise" or "include" or any other variations are intended to contain a non-exclusive inclusion, such that a process, method, product, or equipment comprising a series of elements not only includes the listed elements, but also includes other elements that are not explicitly listed, or elements that are inherent to such a process, method, product, or equipment. An element that is defined by the phrase "comprising a ..." does not exclude the presence of additional elements in the process, method, product, or equipment that comprises the element.

Persons skilled in the art shall understand that, the embodiments of the present disclosure can be provided as a method, a system or a computer program product. Therefore, the present disclosure can adopt the forms of a complete hardware example, a complete software example, or combination of a software example and a hardware example. Moreover, the present description can adopt the form of a computer program product that is implemented on one or more computer-usable storage medium (including but not limited to a disk memory, a CD-ROM, an optical memory, and etc.) including computer-usable program codes.

The present disclosure may be described in the general context of computer executable instructions executed by the computer, e.g., the program module. In general, the program module includes a routine, a program, an object, a component, a data structure, etc. executing a particular task or realizing a particular abstract data type. The present disclosure may also be put into practice in the distributed computing environments where tasks are executed by remote processing devices connected through a communication network. In the distributed computing environments, the program modules may be located in the local and remote computer storage medium including the storage device.

The various embodiments in the disclosure are described in a progressive manner, and the same or similar parts between the various embodiments may be referred to each other, and each embodiment focuses on the differences from the other embodiments. In particular, the system embodiment is simply described since it is substantially similar to the method embodiment, and please refer to the description of the method embodiment for the relevant content.

The above description is merely examples of the embodiments of the present disclosure, and is not intended to limit the present disclosure. Various modifications and variations may be made to the present disclosure by those skilled in the art. Any modifications, equivalents, improvements, etc. made within the spirit and scope of the present disclosure are intended to be included within the scope of the claims of the present application.

## Claims

1. A method for labeling a protection scope of claims of a gene sequence, **characterized in that**, the method is applied to a retrieval engine, the method comprising:
recognizing a gene sequence from claims of a current patent application;
extracting descriptive texts of the gene sequence from the claims based on a preset keyword; and
determining similarity information of the gene sequence based on the extracted descriptive texts, and labeling the protection scope of the claims of the gene sequence according to the similarity information.

2. The method according to claim 1, **characterized in that**, recognizing the gene sequence from the claims of the current patent application comprises:
recognizing a letter sequence consisted of specified letters from the claims of the current patent application, and taking the letter sequence as the gene sequence when a length of the letter sequence reaches a specified length threshold value;
or,
recognizing a sequence identifier from the claims of the current patent application, and reading a gene sequence corresponding to the sequence identifier from a sequence table of the current patent application.

3. The method according to claim 1, **characterized in that**, extracting descriptive texts of the gene sequence from the claims based on the preset keyword comprises:
recognizing from the claims a position where the gene sequence is located, and looking up a length identifier that defines a length of a partial subsequence in the gene sequence from context of the recognized position; and
taking the length identifier as the preset keyword, intercepting a sentence containing the length identifier and the gene sequence, and taking the intercepted sentence as the descriptive texts of the gene sequence.

4. The method according to claim 1, **characterized in that**, determining the similarity information of the gene sequence comprises:
recognizing an effective percentage corresponding to the gene sequence from the descriptive texts, and taking the effective percentage as the similarity information of the gene sequence.

5. The method according to claim 1, **characterized in that**, determining the similarity information of the gene sequence comprises:
recognizing regional information for defining a position of a gene subsequence or a core function sequence in the gene sequence from the descriptive texts, and determining a length of the gene subsequence or the core function sequence according to the regional information; andcalculating a percentage of the length of the gene subsequence or the core function sequence in a total length of the gene sequence, and taking the calculated percentage as the similarity information of the gene sequence.

6. The method according to claim 1, **characterized in that**, labeling the scope of the claims of the gene sequence based on the similarity information comprises:
taking a percentage characterizing the similarity information as a lower limit value of the scope of the claims of the gene sequence.

7. A system for labeling a protection scope of claims of a gene sequence, **characterized by** comprising:
a gene sequence recognition unit for recognizing the gene sequence from the claims of a current patent application;
a descriptive text extraction unit for extracting descriptive texts of the gene sequence from the claims based on a preset keyword; and
a claim scope determination unit for determining similarity information of the gene sequence based on the extracted descriptive texts, and labeling the protection scope of the claims of the gene sequence based on the similarity information.

8. A system for labeling a protection scope of claims of a gene sequence, **characterized by** comprising: a processor and a memory for storing a computer program which, when being executed by the processor, implements the method according to any of claims 1 to 6.

9. A computer storage medium, **characterized in that**, the computer storage medium is used for storing a computer program which, when being executed by a processor, implements the method according to any of claims 1 to 6.

10. A method for retrieving a gene sequence, **characterized by** comprising:
acquiring a gene sequence to be retrieved;
grabbing a text gene sequence from patent applications in a patent library, and comparing the gene sequence to be retrieved with the grabbed text gene sequence, to determine a target gene sequence similar to the gene sequence to be retrieved; and
displaying a retrieval result of patent applications containing the target gene sequence.

11. The method according to claim 10, **characterized in that**, grabbing the text gene sequence from patent applications in the patent library comprises:
recognizing a letter sequence consisted of specified letters from claims of a current patent application, and taking the letter sequence as the text gene sequence when a length of the letter sequence reaches a specified length threshold value.

12. The method according to claim 10, **characterized in that**, grabbing the text gene sequence from patent applications in the patent library comprises:
recognizing a target sequence identifier from claims of a current patent application, and reading a text gene sequence corresponding to the target sequence identifier from a sequence table of the current patent application.

13. The method according to claim 10, **characterized in that**, determining the target gene sequence similar to the gene sequence to be retrieved comprises:
calculating a similarity between the gene sequence to be retrieved and the text gene sequence according to a preset algorithm, and if the calculated similarity is greater than or equal to a specified similarity threshold, taking the text gene sequence as the target gene sequence similar to the gene sequence to be retrieved.

14. The method according to claim 10, **characterized by** further comprising: after determining the target gene sequence similar to the gene sequence to be retrieved,
determining patent applications containing the target gene sequence;
extracting descriptive texts of the target gene sequence from the claims of the patent applications according to a preset keyword; and
determining similarity information of the target gene sequence based on the extracted descriptive texts, and labeling the scope of the claims of the target gene sequence based on the similarity information.

15. The method according to claim 14, **characterized in that**, extracting the descriptive texts of the target gene sequence from the claims of the patent applications according to the preset keyword comprises:
recognizing from the claims a position where the target gene sequence is located, and looking up a length identifier that defines a length of a partial subsequence in the target gene sequence from context of the recognized position; and
taking the length identifier as the preset keyword, intercepting a sentence containing the length identifier and the target gene sequence, and taking the intercepted sentence as the descriptive texts of the target gene sequence.

16. The method according to claim 14, **characterized in that**, determining the similarity information of the target gene sequence comprises:
recognizing a percentage of a similarity variable range to which the target gene sequence corresponds from the descriptive texts, and taking the percentage of the similarity variable range as the similarity information of the target gene sequence;
or,
recognizing regional information for defining a position of a gene subsequence or a core function sequence in the target gene sequence from the descriptive texts, and determining a length of the gene subsequence or the core function sequence according to the regional information; calculating a percentage of the length of the gene subsequence or the core function sequence in a total length of the target gene sequence, and taking the calculated percentage as the similarity information of the target gene sequence.

17. The method according to claim 14, **characterized in that**, labeling the scope of claims of the target gene sequence based on the similarity information comprises:
taking a percentage characterizing the similarity information as a lower limit value of the scope of the claims of the target gene sequence.

18. The method according to claim 10, **characterized by** further comprising: when acquiring the gene sequence to be retrieved, receiving a specified similarity range input by a user; correspondingly, after determining the target gene sequence similar to the gene sequence to be retrieved, the method further comprises:
filtering, from the target gene sequence, a selected gene sequence whose similarity to the gene sequence to be retrieved is within the specified similarity range, and displaying a retrieval result of the patent applications containing the selected gene sequence.

19. The method according to claim 10, **characterized by** further comprising: after determining the target gene sequence similar to the gene sequence to be retrieved,
converting the patent applications containing the target gene sequence into a similarity map based on the similarity between the gene sequence to be retrieved and the target gene sequence; wherein in the similarity map, a distance between a patent application containing the target gene sequence and the gene sequence to be retrieved is inversely proportional to the similarity.

20. The method according to claim 19, **characterized by** further comprising: after converting the patent applications containing the target gene sequence into the similarity map,
receiving a similarity range input by a user and removing a patent application having a similarity outside the similarity range from the similarity map.

21. The method according to claim 19, **characterized by** further comprising: after converting the patent applications containing the target gene sequence into the similarity map,
receiving a similarity range input by a user, determining in the similarity map a first patent application having a similarity within the similarity range, and determining in the similarity map a second patent application having a similarity outside the similarity range; and
displaying the first patent application and the second patent application in different colors in the similarity map.

22. The method according to claim 10, **characterized by** further comprising: after determining the target gene sequence similar to the gene sequence to be retrieved,
constructing the patent applications containing the target gene sequence as a gene sequence development progress map according to information of filing date.

23. The method according to claim 10, **characterized by** further comprising: after determining the target gene sequence similar to the gene sequence to be retrieved,
recognizing a core function sequence from the gene sequence to be retrieved, and selecting patent applications containing the core function sequence; and
constructing the patent applications containing the core function sequence a core sequence development progress map according to information of filing date.

24. A system for retrieving a gene sequence, **characterized by** comprising:
a retrieval information acquisition unit for acquiring a gene sequence to be retrieved;
a target gene sequence retrieval unit for grabbing a text gene sequence from a patent application of a patent library, and comparing the gene sequence to be retrieved with the grabbed text gene sequence, to determine a target gene sequence similar to the gene sequence to be retrieved; and
a retrieval result displaying unit for displaying a retrieval result of a patent application containing the target gene sequence.

25. A system for retrieving a gene sequence, **characterized by** comprising: a processor and a memory for storing a computer program which, when being executed by the processor, implements the method according to any of claims 10 to 23.

26. A computer storage medium, **characterized in that**, the computer storage medium is used to store a computer program which, when being executed by a processor, implements the method according to any of claims 10 to 23.

27. A method for retrieving a gene sequence, **characterized by** comprising:
acquiring a gene sequence to be retrieved and a preset similarity threshold value;
splitting the gene sequence to be retrieved into a plurality of gene subsequences based on the preset similarity threshold value; and
performing a sequence retrieval for each of the gene subsequences in a patent library to determine a target patent application corresponding to the gene subsequence, and outputting a retrieval result of the target patent application.

28. The method according to claim 27, **characterized in that**, splitting the gene sequence to be retrieved into the plurality of gene subsequences based on the preset similarity threshold value comprises:
determining a target length of the split gene subsequence according to the preset similarity threshold value and a length of the gene sequence to be retrieved; and
extracting subsequences conforming to the target length from the gene sequence to be retrieved, and taking the extracted subsequences as the split gene subsequences.

29. The method according to claim 27, **characterized in that**, determining the target patent application corresponding to the gene subsequence comprises:
grabbing a text gene sequence from patent applications in the patent library and determining whether the grabbed text gene sequence contains the gene subsequence; and
if the grabbed text gene sequence contains the gene subsequence, taking a patent application containing the text gene sequence as the target patent application corresponding to the gene subsequence.

30. The method according to claim 29, **characterized in that**, grabbing the text gene sequence from patent applications in the patent library comprises:
recognizing a letter sequence consisted of specified letters from claims of a current patent application, and taking the letter sequence as the text gene sequence when a length of the letter sequence reaches a specified length threshold value.

31. The method according to claim 29, **characterized in that**, grabbing the text gene sequence from the patent applications in the patent library comprises:
recognizing a target sequence identifier from claims of a current patent application, and reading the text gene sequence corresponding to the target sequence identifier from a sequence table of the current patent application.

32. A system for retrieving a gene sequence, **characterized by** comprising:
a retrieval information acquisition unit for acquiring a gene sequence to be retrieved and a preset similarity threshold value;
a subsequence splitting unit for splitting the gene sequence to be retrieved into a plurality of gene subsequences based on the preset similarity threshold value; and
a retrieval result outputting unit for performing a sequence retrieval for each of the gene subsequences in a patent library to determine target patent applications corresponding to the gene subsequences, and outputting a retrieval result of the target patent applications.

33. A system for retrieving a gene sequence, **characterized by** comprising: a processor and a memory for storing a computer program which, when being executed by the processor, implements the method according to any of claims 27 to 31.

34. A computer storage medium, **characterized in that**, the computer storage medium is used for storing a computer program which, when being executed by a processor, implements the method according to any of claims 27 to 31.

35. A method for labeling infringement risk information of a gene sequence, **characterized in that**, the method is applied to a retrieval engine, the method comprising:
acquiring a gene sequence to be retrieved, and querying patent applications containing a target gene sequence similar to the gene sequence to be retrieved;
extracting descriptive texts containing the target gene sequence from the claims of the patent applications, and determining a scope of the claims of the target gene sequence based on the descriptive texts;
determining a similarity between the gene sequence to be retrieved and the target gene sequence, and comparing the similarity with the scope of claims of the target gene sequence; and
labeling infringement risk information of the patent applications with respect to the gene sequence to be retrieved based on a comparison result.

36. The method according to claim 35, **characterized in that**, extracting descriptive texts containing the target gene sequence from the claims of the patent applications comprises:
determining a sequence identifier of the target gene sequence in the claims from a sequence table of the patent applications, and recognizing from the claims a position where the sequence identifier is located;
looking up a length identifier that defines a length of a partial subsequence in the target gene sequence from the context of the recognized position; and
intercepting a sentence containing the length identifier and the sequence identifier, and taking the intercepted sentence as the descriptive texts containing the target gene sequence.

37. The method according to claim 35, **characterized in that**, determining the scope of the claims of the target gene sequence based on the descriptive texts comprises:
recognizing a percentage of a similarity variable range to which the target gene sequence corresponds from the descriptive texts, and taking the percentage of the similarity variable range as a lower limit value of the scope of the claims of the target gene sequence.

38. The method according to claim 35, **characterized in that**, determining the scope of the claims of the target gene sequence based on the descriptive texts comprises:
recognizing regional information for defining a position of a gene subsequence or a core function sequence in the target gene sequence from the descriptive texts, and determining a length of the gene subsequence or the core function sequence according to the regional information; andcalculating a percentage of the length of the gene subsequence or the core function sequence in a total length of the target gene sequence, and taking the calculated percentage as a lower limit value of the scope of claims of the target gene sequence.

39. The method according to claim 35, **characterized by** further comprising: when acquiring the gene sequence to be retrieved,
receiving a specified similarity range input by a user; correspondingly, a similarity between the target gene sequence contained in the patent applications obtained by query and the gene sequence to be retrieved is within the specified similarity range.

40. The method according to claim 35, **characterized by** further comprising: after querying the patent applications matching the gene sequence to be retrieved, converting the patent applications obtained by query into a similarity map according to the similarity between the target gene sequence in the patent applications and the gene sequence to be retrieved; wherein in the similarity map, a distance between the patent application and the gene sequence to be retrieved is inversely proportional to the similarity.

41. The method according to claim 40, **characterized by** further comprising: after converting the patent application obtained by query into the similarity map, receiving a similarity range input by a user and removing a patent application having a similarity outside the similarity range from the similarity map.

42. The method according to claim 40, **characterized by** further comprising: after converting the patent application obtained by query into the similarity map,
receiving a similarity range input by a user, determining in the similarity map a first patent application having a similarity within the similarity range, and determining in the similarity map a second patent application having a similarity outside the similarity range; and
displaying the first patent application and the second patent application in different colors in the similarity map.

43. The method according to claim 35, **characterized by** further comprising: after querying the patent applications matching the gene sequence to be retrieved,
constructing the patent applications obtained by query as a gene sequence development progress map according to information of filing date.

44. The method according to claim 35, **characterized by** further comprising: after querying the patent applications matching the gene sequence to be retrieved,
recognizing a core function sequence from the gene sequence to be retrieved, and filtering target patent applications containing the core function sequence from the patent applications obtained by query; and
constructing the target patent applications as a core sequence development progress map according to information of filing date.

45. A system for labeling infringement risk information of a gene sequence, **characterized in that**, the system comprises:
a patent application querying unit for acquiring a gene sequence to be retrieved, and querying patent applications containing a target gene sequence similar to the gene sequence to be retrieved;
a claim scope determination unit for extracting descriptive texts containing the target gene sequence from the claims of the patent application, and determining a scope of claims of the target gene sequence based on the descriptive texts;
a comparison unit for determining a similarity between the gene sequence to be retrieved and the target gene sequence, and comparing the similarity with the scope of the claims of the target gene sequence; and
a risk judgment unit for labeling infringement risk information of the patent applications with respect to the gene sequence to be retrieved, based on a comparison result.

46. A system for labeling infringement risk information of a gene sequence, **characterized by** comprising: a processor and a memory for storing a computer program which, when being executed by the processor, implements the method according to any of claims 35 to 44.

47. A computer storage medium, **characterized in that**, the computer storage medium is used for storing a computer program which, when being executed by a processor, implements the method according to any of claims 35 to 44.
